# EUROPEAN PATENT APPLICATION

(11) **EP 2 891 719 A1**
(43) Date of publication of application: **08.07.2015**
(21) Application number: 12883595.6
(22) Date of filing: 28.08.2012
(51) Int. Cl.: C12Q 1/68

(54) **TEST COMPOSITION FOR SCREENING CANCERS**

(71) Applicant: Istat Biomedical Co., Ltd., 22102 New Taipei City (TW)
(72) Inventor: CHANG, Chi-Feng, 22102 New Taipei City (TW); WANG, Huei-Jen, 22102 New Taipei City (TW); LIOU, Yu-Ligh, 22102 New Taipei City (TW)
(74) Representative: Izquierdo Faces, José
(86) International application number: PCT/CN2012/080655
(87) International publication number: WO 2014/032227

(57) **Abstract**

Provided in the present invention are a molecule for biomarking cancers and a test composition for screening cancers and a detection method thereof comprising designing a number of oligonucleotide primers or probes by analyzing specimens for methylated regions of targeted genes PAX1, ZNF582, SOX1 and NKX6-1 in physical examination, and then using the oligonucleotide probes to detect whether methylation exists in the target genes, and further judge the likelihood of the occurrence of cancer. The detection methods for methylation status include methylation-specific PCR, (MSP), quantitative methylation-specific PCR (QMSP), bisulfite sequencing (BS), microarrays, mass spectrometer, denaturing high-performance liquid chromatography (DHPLC), pyrosequencing, and enzyme-linked immunosorbent assay (ELISA).

SEQUENCE LISTING

<110> iState Biomedical Co.,Ltd.

<120> Cancer Detection Kit

<130> IP120842

<160> 80

<210> 1
<211> 21
<212> DNA
<213> artificial sequence
<220>
<223> primer or probe

<400> 1
attcgcgcgt tttcggcgtg a 21

<210> 2
<211> 26
<212> DNA
<213> artificial sequence
<220>
<223> primer or probe

<400> 2
gttaaattga ttttcgtacg ttgtag 26

<210> 3
<211> 20
<212> DNA
<213> artificial sequence
<220>
<223> primer or probe

<400> 3
tattttgggt ttggggtcgc 20

<210> 4
<211> 22
<212> DNA
<213> artificial sequence
<220>
<223> primer or probe

<400> 4
ttattttggg tttggggtcg cg 22

<210> 5
<211> 20
<212> DNA
<213> artificial sequence
<220>
<223> primer or probe

<400> 5
gggcggtagc gcgtttcgtt 20

<210> 6
<211> 23
<212> DNA
<213> artificial sequence
<220>
<223> primer or probe

<400> 6
tagcggcggc ggtaggtttt gga                                               23


<210> 7
<211> 22
<212> DNA
<213> artificial sequence
<220>
<223> primer or probe

<400> 7
gtagtgacgg gaattaatga gt                                                22


<210> 8
<211> 20
<212> DNA
<213> artificial sequence
<220>
<223> primer or probe

<400> 8
aacatcccac gaccacgccg                                                   20


<210> 9
<211> 20
<212> DNA
<213> artificial sequence
<220>
<223> primer or probe

<400> 9
acgaccacgc cgaaaaccgt                                                   20


<210> 10
<211> 21
<212> DNA
<213> artificial sequence
<220>
<223> primer or probe

<400> 10
acaaacaacg aaaaatacgc g                                                 21


<210> 11
<211> 21
<212> DNA
<213> artificial sequence
<220>
<223> primer or probe

<400> 11
acgacgaaaa aaacgacgac g                                                21


<210> 12
<211> 25
<212> DNA
<213> artificial sequence
<220>
<223> primer or probe

<400> 12
ttaaattgat tttcgtacgt tgtag                                            25


<210> 13
<211> 20
<212> DNA
<213> artificial sequence
<220>
<223> primer or probe

<400> 13
gcgaccccaa acccaaaata                                                  20


<210> 14
<211> 19
<212> DNA
<213> artificial sequence
<220>
<223> primer or probe

<400> 14
ctcccaaaac actctccac                                                   19


<210> 15
<211> 2
<212> DNA
<213> artificial sequence
<220>
<223> primer or probe

<400> 15
agtagcggcg gcggtaggtt                                                  20


<210> 16
<211> 20
<212> DNA
<213> artificial sequence
<220>
<223> primer or probe

<400> 16
aacgaaacgc gctaccgccc                                                  20


<210> 17
<211> 21
<212> DNA

<213> artificial sequence
<220>
<223> primer or probe

<400> 17
cgcgacccca aacccaaaat a                                                 21


<210> 18
<211> 22
<212> DNA
<213> artificial sequence
<220>
<223> primer or probe

<400> 18
aaaacactct ccacgcccgc ga                                                22


<210> 19
<211> 17
<212> DNA
<213> artificial sequence
<220>
<223> primer or probe

<400> 19
attgattttc gtacgtt                                                      17


<210> 20
<211> 20
<212> DNA
<213> artificial sequence
<220>
<223> primer or probe

<400> 20
aacctaccgc cgccgctact                                                   20


<210> 21
<211> 21
<212> DNA
<213> artificial sequence
<220>
<223> primer or probe

<400> 21
cctcccaaaa cactctccac g                                                 21


<210> 22
<211> 22
<212> DNA
<213> artificial sequence
<220>
<223> primer or probe

<400> 22
cccgaaaacc gaaaaccg                                                     22

<210> 23
<211> 21
<212> DNA
<213> artificial sequence
<220>
<223> primer or probe

<400> 23
acgcccgaaa accgaaaacc g                                                21


<210> 24
<211> 21
<212> DNA
<213> artificial sequence
<220>
<223> primer or probe

<400> 24
atcgcccgcc ccttacccat a                                                21


<210> 25
<211> 22
<212> DNA
<213> artificial sequence
<220>
<223> primer or probe

<400> 25
cctacctatc gcccgcccct ta                                               22


<210> 26
<211> 22
<212> DNA
<213> artificial sequence
<220>
<223> primer or probe

<400> 26
acgatttacg cggagttaga ag                                               22


<210> 27
<211> 28
<212> DNA
<213> artificial sequence
<220>
<223> primer or probe

<400> 27
tgacggtttt ttgtttattc ggttattc                                         28


<210> 28
<211> 30
<212> DNA
<213> artificial sequence
<220>
<223> primer or probe

<400> 28

agtgacggtt ttttgtttat tcggttattc 30

<210> 29
<211> 23
<212> DNA
<213> artificial sequence
<220>
<223> primer or probe

<400> 29
cggagggata ttgcggcgtc ggt 23

<210> 30
<211> 19
<212> DNA
<213> artificial sequence
<220>
<223> primer or probe

<400> 30
atgggaacgt aacggatga 19

<210> 31
<211> 20
<212> DNA
<213> artificial sequence
<220>
<223> primer or probe

<400> 31
atttaacgat ttacgcggag 20

<210> 32
<211> 23
<212> DNA
<213> artificial sequence
<220>
<223> primer or probe

<400> 32
aaacgtacct acgcaatacg cga 23

<210> 33
<211> 21
<212> DNA
<213> artificial sequence
<220>
<223> primer or probe

<400> 33
cgaacgcaaa cgtacctacg c 21

<210> 34
<211> 24
<212> DNA
<213> artificial sequence
<220>

<223> primer or probe

<400> 34
accgaacgca aacgtaccta cgca                                              24


<210> 35
<211> 21
<212> DNA
<213> artificial sequence
<220>
<223> primer or probe

<400> 35
acccaaaacg cgcttccacc a                                                 21


<210> 36
<211> 17
<212> DNA
<213> artificial sequence
<220>
<223> primer or probe

<400> 36
cgaataaccg aataaac                                                      17


<210> 37
<211> 15
<212> DNA
<213> artificial sequence
<220>
<223> primer or probe

<400> 37
tacgcgaaaa aatac                                                         15


<210> 38
<211> 16
<212> DNA
<213> artificial sequence
<220>
<223> primer or probe

<400> 38
cgccgtacgc aaccga                                                        16


<210> 39
<211> 22
<212> DNA
<213> artificial sequence
<220>
<223> primer or probe

<400> 39
atttcaaaat aaaaccgaac gc                                                22


<210> 40
<211> 20

<212> DNA
<213> artificial sequence
<220>
<223> primer or probe

<400> 40
acccgacctt aaaaccgaat                                                    20


<210> 41
<211> 25
<212> DNA
<213> artificial sequence
<220>
<223> primer or probe

<400> 41
gcgttttttt tttttcgtta ttggc                                              25


<210> 42
<211> 27
<212> DNA
<213> artificial sequence
<220>
<223> primer or probe

<400> 42
tgcgtttttt ttttttcgtt attggcg                                            27


<210> 43
<211> 20
<212> DNA
<213> artificial sequence
<220>
<223> primer or probe

<400> 43
cgcggcgcgt cgttttgtta                                                    20


<210> 44
<211> 19
<212> DNA
<213> artificial sequence
<220>
<223> primer or probe

<400> 44
tggaggtcgt tgaggatcg                                                     19


<210> 45
<211> 20
<212> DNA
<213> artificial sequence
<220>
<223> primer or probe

<400> 45
cggcggtcgg cgaggagata                                                    20

<210> 46
<211> 20
<212> DNA
<213> artificial sequence
<220>
<223> primer or probe

<400> 46
gcgttttcgt ttcgagcgta                                                    20


<210> 47
<211> 20
<212> DNA
<213> artificial sequence
<220>
<223> primer or probe

<400> 47
aggatcgagc gtaggaggaa                                                    20


<210> 48
<211> 23
<212> DNA
<213> artificial sequence
<220>
<223> primer or probe

<400> 48
cgcgctatct ccttcctcct acg                                                23


<210> 49
<211> 19
<212> DNA
<213> artificial sequence
<220>
<223> primer or probe

<400> 49
gccgctacgc gctatctcc                                                     19


<210> 50
<211> 20
<212> DNA
<213> artificial sequence
<220>
<223> primer or probe

<400> 50
gcaacccaaa cgccctcgac                                                    20


<210> 51
<211> 21
<212> DNA
<213> artificial sequence
<220>
<223> primer or probe

<400> 51
cgatacgcta aacccgaccc g                                                21

<210> 52
<211> 20
<212> DNA
<213> artificial sequence
<220>
<223> primer or probe

<400> 52
cgcggcgcgt cgttttgtta                                                  20

<210> 53
<211> 19
<212> DNA
<213> artificial sequence
<220>
<223> primer or probe

<400> 53
cgatcctcaa cgacctcca                                                   19

<210> 54
<211> 21
<212> DNA
<213> artificial sequence
<220>
<223> primer or probe

<400> 54
cgatacgcta aacccgaccc g                                                21

<210> 55
<211> 20
<212> DNA
<213> artificial sequence
<220>
<223> primer or probe

<400> 55
gctcgatcct caacgacctc                                                  20

<210> 56
<211> 20
<212> DNA
<213> artificial sequence
<220>
<223> primer or probe

<400> 56
acgatcgaaa tcgccgtctt                                                  20

<210> 57
<211> 21
<212> DNA
<213> artificial sequence

<220>
<223> primer or probe

<400> 57
tgtcgttttt cgcgtggagg g                                                21


<210> 58
<211> 20
<212> DNA
<213> artificial sequence
<220>
<223> primer or probe

<400> 58
cgtggtcgtg ggatgttagc                                                  20


<210> 59
<211> 24
<212> DNA
<213> artificial sequence
<220>
<223> primer or probe

<400> 59
tcggcgtggt cgtgggatgt tagc                                             24


<210> 60
<211> 20
<212> DNA
<213> artificial sequence
<220>
<223> primer or probe

<400> 60
acggttttcg gcgtggtcgt                                                  20


<210> 61
<211> 19
<212> DNA
<213> artificial sequence
<220>
<223> primer or probe

<400> 61
ttcgggcgcg tcgagtgtt                                                   19


<210> 62
<211> 20
<212> DNA
<213> artificial sequence
<220>
<223> primer or probe

<400> 62
aacatcccac gaccacgccg                                                  20


<210> 63

<211> 20
<212> DNA
<213> artificial sequence
<220>
<223> primer or probe

<400> 63
acgaccacgc cgaaaaccgt                                                    20


<210> 64
<211> 21
<212> DNA
<213> artificial sequence
<220>
<223> primer or probe

<400> 64
acaaacaacg aaaaatacgc g                                                  21


<210> 65
<211> 23
<212> DNA
<213> artificial sequence
<220>
<223> primer or probe

<400> 65
gacaaacaac gaaaaatacg cga                                                23


<210> 66
<211> 21
<212> DNA
<213> artificial sequence
<220>
<223> primer or probe

<400> 66
acgacgaaaa aaacgacgac g                                                  21


<210> 67
<211> 19
<212> DNA
<213> artificial sequence
<220>
<223> primer or probe

<400> 67
cgctaccgaa aattactcg                                                     19


<210> 68
<211> 18
<212> DNA
<213> artificial sequence
<220>
<223> primer or probe

<400> 68
cgaataccct ccattacc                                                      18

<210> 69
<211> 21
<212> DNA
<213> artificial sequence
<220>
<223> primer or probe

<400> 69
acaaacaacg aaaaatacgc g                                                21


<210> 70
<211> 19
<212> DNA
<213> artificial sequence
<220>
<223> primer or probe

<400> 70
aacactcgac gcgcccgaa                                                   19


<210> 71
<211> 25
<212> DNA
<213> artificial sequence
<220>
<223> primer or probe

<400> 71
agggttattt tgaaaaggga gatat                                            25


<210> 72
<211> 25
<212> DNA
<213> artificial sequence
<220>
<223> primer or probe

<400> 72
ttttaagggg aagatgggat agaag                                            25


<210> 73
<211> 23
<212> DNA
<213> artificial sequence
<220>
<223> primer or probe

<400> 73
agaggtgggg ataggtattg ggt                                              23


<210> 74
<211> 18
<212> DNA
<213> artificial sequence
<220>
<223> primer or probe

<400> 74
cttctatccc atcttccc                                                    18

<210> 75
<211> 20
<212> DNA
<213> artificial sequence
<220>
<223> primer or probe

<400> 75
ttcattctaa cccaatacct                                                  20

<210> 76
<211> 21
<212> DNA
<213> artificial sequence
<220>
<223> primer or probe

<400> 76
tgttagagta aagtatagag t                                                21

<210> 77
<211> 23
<212> DNA
<213> artificial sequence
<220>
<223> primer or probe

<400> 77
aacccaatac ctatccccac ctc                                              23

<210> 78
<211> 27
<212> DNA
<213> artificial sequence
<220>
<223> primer or probe

<400> 78
aacaattata aactccaacc accaaac                                         27

<210> 79
<211> 25
<212> DNA
<213> artificial sequence
<220>
<223> primer or probe

<400> 79
actccaacca ccaaaccttc attct                                            25

<210> 80
<211> 20
<212> DNA

<213> artificial sequence
<220>
<223> primer or probe

<400> 80
accgacccca ctaatacccg                                                    20

## Description

### BACKGROUND OF THE INVENTION

### 1. FIELD OF THE INVENTION

Present invention relates to cancer biomarkers and a cancer screening test composition for determination of the occurrence of cancer based on the presence of methylated target genes by designing several target gene-specific oligonucleotide primers or probes and analyzing the methylated sites of the target genes in samples.

### 2. DESCRIPTION OF THE PRIOR ART

According to a 2008 WHO survey, cancer is the one of the top ten leading causes of death worldwide and among which lung cancer, liver cancer, stomach cancer, colorectal cancer, and oral cancer showed higher mortality rates in men, while breast cancer, lung cancer, cervical cancer and colorectal cancer ranked the top in women. Consequently, researchers worldwide have devoted in studying the methods for early detection or for prediction of cancer outcomes. Moreover, early detection of most cancers significantly increases the cure rates. Conventional methods for cancer detection such as X-ray, smear, tumor markers in the blood and ultrasound have cancer screening rates between 0.2% and 0.3%, and the screening rate will increase to 0.5% if gastrointestinal endoscopy is also performed. Nonetheless, the data mentioned above remains insufficient to convince the public that regular health exams can effectively detect cancer at an early stage. Furthermore, these tests and judgments usually are time-consuming, inconvenience, and with low screening rates.

Up to date, epigenetic modifications have been widely studied and the importance of epigenetics on alteration of gene expression as well as induction of tumorigenesis has also been proved (Ting et al.,2006), and among which DNA methylation is one mechanism. The message that affects phenotype may be stored in the modified 5-methylcytosine and 5-methylcytosine was found in the palindromic sequence 5'-CpG-3' in the mammalian cells. In mammalian cells, except the so-called "CpG islands" (CGIs) regions, most CpG dinucleotides are methylated. The CpG island refers to a significant number of GC- and CpG-within a region containing approximately 1,000 bp (1 Kb) and is usually located in the nearby areas of genes and around the promoters of widely expressed genes. Methylation of cytosine occurs after DNA synthesis and the methyl donor S-adenosylmethionine (SAM) transfers a methyl group to the 5th carbon of cytosine through an enzymatic reaction catalyzed by DNA methyltransferase (DNMTs). DNMT1 is the main methyltransferase in mammalians and is responsible for post-replicative restoration that adds methyl groups to hemimethylated sites and produces methylated DNA and is called maintenance methylation. On the other hand, DNMT3A and DNMT3B are considered mainly responsible for methylation of new sites of DNA and involved in the process called de novo methylation. Loss of methylation of CpG dinucleotides, i.e. low methylation, is the first epigenetic abnormality in cancer cells. However, studies conducted in the past few years indicate that site-specific hypermethylation (e.g. certain tumor suppressor genes) is possibly associated with loss of gene functions and may provide selective advantages during tumorigenesis. Hypermethylation of the CpG islands within the promoter regions can cause chromatin remodeling by gene silencing resulted from histone modifications. In addition to chromosome deletion and gene mutation, epigenetic silencing of the tumor suppressor genes through hypermethylation of the promoters are also very common in human cancers (Estelle et al., 1999; Herman et al., 2003).

Recent epidemiological studies have shown that the concentration of serum folate (a major source of methyl groups) is correlated to infection and clearance of HPV. In the metabolic process of the methyl cycle, genetic polymorphisms of the enzymes have also been reported to associated with development of cervical intraepithelial lesions. As the concept of epigenetic evolution, many studies have demonstrated the correlation between DNA methylation and cervical cancer, and relevant studies have increased significantly which prompts the possibility of using methylation as a screening method for cervical cancer. Due to the characteristics of the interactions between genetics and environment, the degree of methylation of the tumor suppressor genes vary from gene to gene and from population to population, and different diseases will have different methylator phenotypes; however, the methylator phenotype of cervical cancer and its association with HPV genotypes remain unclear. Moreover, which specific genes are methylated in cervical cancer as well as how many genes are required before they can meet the needs of clinical applications are still issues need to be confirmed in the future.

Dr. Hung-Cheng Lai at the National Defense Medical Center had previously disclosed related inventions, different techniques and methods in prior art, including patent applications filed in Taiwan (TW Pat. Pub. No. 200831900, TW Pat. Pub. No. 201038739), China (CN Appl. No. 200810094659.2, CN Appl. No. 200910135501.X), Malaysia (U120085354) and the United States (US Pat. Pub. No. 20080311570, US Pat. Pub. No. 20110045465) (thereinafter referred to as prior applications). Though theoretical development of detection of gene methylation has been continued for a while and been widely used by researchers, its application in clinical-related fields such as medical detection requires more studies on the stability and reproducibility. Also, detection of methylated genes is not a widely used method in clinical practice currently and this is partially due to the needs of significant amount of research and related verification to support the clinical significance of genes. Additionally, how to provide stable test methods also presents considerable technical obstacles.

The inventor of the present invention understands the insufficiency and defects of the prior art and finally completed the invention and developed a method and a test composition that can be used to screen for a variety of cancers after years of painstaking research. The invention allows not only detection of methylation of the related genes but industrial application, for example, define the related sequences of target genes and their concentration ranges. Moreover, the invention also provides more information on verification and support in the cancer field, makes detection of target genes more reproducible, and allows medical applications. The test composition for cancer screening of the present invention is more accurate and is designed in the form of a test composition for faster, more convenient, and more efficient results and is a complete product for cancer screening.

### SUMMARY OF THE INVENTION

In one aspect, present invention provides a test composition for determination of methylation of CpG sequences of the target genes in a test sample, that is, extraction of gDNA from the test sample first and the extracted gDNA is then subjected to appropriate pre-treatments and chemical reactions so as to allow detection of gene methylation in the target genes by using this test composition. The test composition is comprising of:
(1) a gene-specific primer mixture at a concentration between 20 nM and 1250 nM and the mixture is consisting of at least one forward primer and one reverse primer, the sequences of the forward primer and reverse primer are selected from the nucleotide sequences that share at least 80% homology with the sequences indicated in SEQ ID No: 1-70, or is one or more sequences containing at least 10 continuous nucleotides identical to the sequences indicated in SEQ ID No: 1-70, and the best sequence is the one contains the identical nucleotides;
(2) a nucleic acid molecule test mixture for internal control genes at a concentration between 20 nM and 1250 nM and the test mixture is comprising of at least one forward primer and one reverse primer;
(3) a master mixture for PCR and the master mixture is comprising of at least polymerase, dNTPs, and Magnesium.

According to the invention, the nucleic acid molecule test mixture for internal control genes included in the test composition comprises forward primers and reverse primers and the sequences of said primers are also selected from the nucleotide sequences that share at least 80% homology with the sequences indicated in SEQ ID No: 71-80, or is one or more sequences containing at least 10 continuous nucleotides identical to the sequences indicated in SEQ ID No: 71-80, and the best sequence is the one contains the identical nucleotides. Wherein the primers for the target genes and the internal control genes can be mixed together for preparation of the nucleic acid molecule test mixture in a single tube.

According to the invention, the gene-specific primer mixture further contains probes and the sequences of the probes are selected from the nucleotide sequences that share at least 80% homology with the sequences indicated in SEQ ID No : 1-70, or is one or more or their complementary sequences containing at least 10 continuous nucleotides identical to the sequences indicated in SEQ ID No: 1-70, and the best sequence is the one contains the identical nucleotides; the nucleic acid molecule test mixture for internal control genes further contains the probes that are capable of detection of the amplified products of the internal control genes. In another aspect, present invention also provides the primers of the internal control genes and the sequences of the forward and reverse primers are selected from the nucleotide sequences that share at least 80% homology with the sequences indicated in SEQ ID No: 71-80, or is one or more sequences containing at least 10 continuous nucleotides identical to the sequences indicated in SEQ ID No: 71-80, and the best sequence is the one contains the identical nucleotides; the sequences of the probes are selected from the nucleotide sequences that share at least 80% homology with the sequences indicated in SEQ ID No: 71-80, or is one or more or the complementary sequences containing at least 10 continuous nucleotides identical to the sequences indicated in SEQ ID No: 71-80, and the best sequence is the one contains the identical nucleotides. Wherein the primers of the target genes and the internal control genes can be mixed together for preparation of the nucleic acid molecule test mixture in a single tube.

According to the invention, the master mixture for PCR further contains a fluorescent substance that can identify the amplified PCR products or a detectable substance that can react with double-stranded DNA and produce fluorescence, said substance includes the SYBR series, e.g. SYBER Green and Syber Gold.

According to the invention, the internal control gene of the test sample is at least one or more than one of the genes selected from the following group: Col2A, ß-Globin, GAPDH (glyceraldehyde-3-phosphate dehydrogenase), and ß-actin.

According to the invention, the probes for the target genes and internal control genes in the test composition are labeled with a fluorescent dye and said label is selected from the group comprising of the following fluorescent dyes: FAM, HEX, TET, TAMRA, Cy3, Cy5, Cy5.5, VIC, Red610, Yellow 555, Texas Red, Yakima Yellow, BHQ-1, BHQ-2, and BHQ-3.

According to the invention, the genes in the test sample are selected from at least one or more of the following genes: PAX1, ZNF582, SOX1 and NKX6-1.

According to the invention, the test composition can be further used for detection of abnormal cell proliferation. Wherein the abnormal cell proliferation includes precancerous lesions, tumor detection, detection of tumor recurrence, prediction of the effect of cancer drug, or detection of cancer outcomes. Wherein the malignant tumor refers to one of the following cancers: cervical cancer, oral cancer, head and neck cancer, esophageal cancer, colorectal cancer, liver cancer, ovarian cancer, breast cancer, tongue cancer, lung adenocarcinoma, and skin cancer.

According to the invention, the identification methods for amplified PCR products include fluorescence, sequencing, microarrays, mass spectrometer, denaturing high-performance liquid chromatography (DHPLC), pyrosequencing, or immunoassay.

The term "test sample" refers to samples isolated from the body and said samples include Pap smear, ascites, blood, urine, feces, sputum, oral mucosa epithelial cells, gastric juice, bile, cervical epithelial cells, or cancer tissues collected after surgery.

### DETAILED DESCRIPTION OF THE PREFERRED EMBODIMENT

Present invention will be better elucidated when read in conjunction with the following examples.

In one aspect, present invention provides a test composition for examination of the methylated CpG islands, that is, extraction of gDNA followed by appropriate pre-treatments and chemical reactions, and gene methylation of the treated gDNA can then be examined by using the test composition disclosed in the invention. The test composition is comprising of:
(1) a gene-specific primer mixture at a concentration between 20 nM and 1250 nM and the mixture is consisting of at least one forward primer and one reverse primer, the sequences of the forward primer and reverse primer are selected from the nucleotide sequences that share at least 80% homology with the sequences indicated in SEQ ID No: 1-70, or is one or more sequences containing at least 10 continuous nucleotides identical to the sequences indicated in SEQ ID No: 1-70, and the best sequence is the one contains the identical nucleotides;
(2) a nucleic acid molecule test mixture for internal control genes at a concentration between 20 nM and 1250 nM and the test mixture is comprising of at least one forward primer and one reverse primer;
(3) a master mixture for PCR and the master mixture is comprising of at least polymerase, dNTPs, and Magnesium.

According to the invention, the nucleic acid molecule test composition for internal control genes included in the test composition comprises a forward primer and a reverse primer, wherein the sequences of said primers are selected from the nucleotide sequences that share at least 80% homology with the sequences indicated in SEQ ID No: 71-80 or is one or more sequences containing at least 10 continuous nucleotides identical to the sequences indicated in SEQ ID No:71-80, and the best sequence is the one contains the identical nucleotides. Wherein the primers for target genes and the internal control genes can be mixed together for preparation of the nucleic acid molecule test mixture in a single tube.

According to the invention, the target gene-specific primer mixture further contains probes and the sequences of the probes are selected from the nucleotide sequences that share at least 80% homology with the sequences indicated in SEQ ID No: 1-70, or is one or more or complementary sequences containing at least 10 continuous nucleotides identical to the sequences indicated in SEQ ID No: 1-70, and the best sequence is the one contains the identical nucleotides; the nucleic acid molecule test mixture for internal control genes further contains the probes capable of detection of the amplified products of the internal control genes. In another aspect, present invention also provides the primers for the internal control genes and the sequences of the forward and reverse primers are selected from the nucleotide sequences that share at least 80% homology with the sequences indicated in SEQ ID No: 71-80, or is one or more sequences containing at least 10 continuous nucleotides identical to the sequences indicated in SEQ ID No: 71-80, and the best sequence is the one contains the identical nucleotides; the sequences of the probes are selected from the nucleotide sequences that share at least 80% homology with the sequences indicated in SEQ ID No: 71-80, or is one or more or the complementary sequences containing at least 10 continuous nucleotides identical to the sequences indicated in SEQ ID No: 71-80, and the best sequence is the one contains the identical nucleotides. Wherein the primers for the target genes and internal control genes can be mixed together for preparation of the nucleic acid molecule test mixture in a single tube.

According to the invention, the master mixture for PCR further contains a fluorescent substance that can identify the amplified PCR products or a detectable substance that can react with double-stranded DNA and produce fluorescence, said substance includes the SYBR series, e.g. SYBER Green and Syber Gold.

According to the invention, the internal control genes in the test sample are selected from at least one or more of the following genes: Col2A, ß -Globin, GAPDH (glyceraldehyde-3-phosphate dehydrogenase), and ß-actin.

According to the invention, the probes for the target genes and internal control genes in the test composition are labeled with a fluorescent dye and said label is selected from the group comprising of the following fluorescent dyes: FAM, HEX, TET, TAMRA, Cy3, Cy5, Cy5.5, VIC, Red610, Yellow 555, Texas Red, Yakima Yellow, BHQ-1, BHQ-2, and BHQ-3.

According to the invention, the genes in the test sample are selected from at least one or more of the following genes: PAX1, ZNF582, SOX1 and NKX6-1.

According to the invention, the test composition can be further used for detection of abnormal cell proliferation. Wherein the abnormal cell proliferation includes precancerous lesions, tumor detection, detection of tumor recurrence, prediction of the effect of cancer drug, or detection of cancer outcomes. Wherein the malignant tumor refers to one of the following cancers: cervical cancer, oral cancer, head and neck cancer, esophageal cancer, colorectal cancer, liver cancer, ovarian cancer, breast cancer, tongue cancer, lung adenocarcinoma, and skin cancer.

According to the invention, the identification methods for amplified PCR product include fluorescence, sequencing, microarrays, mass spectrometer, denaturing high-performance liquid chromatography (DHPLC), pyrosequencing, or immunoassay.

Following examples, including materials and methods, are provided herein for further demonstration of the technical features and advantages of the invention; however, it should be understood that the invention is not limited to the preferred embodiments shown. Many changes and modifications in the described embodiments of the invention can, of course, be carried out without departing from the scope thereof.

### Example 1 Materials and Methods

First, DNA was extracted from the test sample and treated with bisulfite. Use the primer sets and probes (see Table 1 to Table 5) disclosed in Table 1 to Table 4 for amplification of PAX1, ZNF582, SOX1 and NKX6-1 as well as the internal control genes for detection. The main components of the test composition are shown in Table 6:

**Table 1 Primer sets and probes used for amplification of the methylation sites of the target gene PAX1**

| ID No : | primer sets and probes for PAX1 |
|---|---|
| SEQ ID No : 1 | 5' attcgcgcgttttcggcgtga 3' |
| SEQ ID No : 2 | 5' gttaaattgattttcgtacgttgtag 3' |
| SEQ ID No : 3 | 5' tattttgggtttggggtcgc 3' |
| SEQ ID No : 4 | 5' ttattttgggtttggggtcgcg 3' |
| SEQ ID No : 5 | 5' gggcggtagcgcgtttcgtt 3' |
| SEQ ID No : 6 | 5' tagcggcggcggtaggttttgga 3' |
| SEQ ID No : 7 | 5' gtagtgacgggaattaatgagt 3' |
| SEQ ID No : 8 | 5' aacatcccacgaccacgccg 3' |
| SEQ ID No : 9 | 5' acgaccacgccgaaaaccgt 3' |
| SEQ ID No : 10 | 5' acaaacaacgaaaaatacgcg 3' |
| SEQ ID No : 11 | 5' acgacgaaaaaaacgacgacg 3' |
| SEQ ID No : 12 | 5' ttaaattgattttcgtacgttgtag 3' |
| SEQ ID No : 13 | 5' gcgaccccaaacccaaaata 3' |
| SEQ ID No : 14 | 5' ctcccaaaacactctccac 3' |
| SEQ ID No : 15 | 5' agtagcggcggcggtaggtt 3' |
| SEQ ID No : 16 | 5' aacgaaacgcgctaccgccc 3' |
| SEQ ID No : 17 | 5' cgcgaccccaaacccaaaata 3' |
| SEQ ID No : 18 | 5' aaaacactctccacgcccgcga 3' |
| SEQ ID No : 19 | 5' attgattttcgtacgtt 3' |
| SEQ ID No : 20 | 5' aacctaccgccgccgctact 3' |
| SEQ ID No : 21 | 5' cctcccaaaacactctccacg 3' |
| SEQ ID No : 22 | 5' cccgaaaaccgaaaaccg 3' |
| SEQ ID No : 23 | 5' acgcccgaaaaccgaaaaccg 3' |
| SEQ ID No : 24 | 5' atcgcccgccccttacccata 3' |
| SEQ ID No : 25 | 5' cctacctatcgcccgcccctta 3' |

**Table 2 Primer sets and probes used for amplification of the methylation sites of the target gene ZNF582**

| ID No : | primer sets and probes for ZNF582 |
|---|---|
| SEQ ID No : 26 | 5' acgatttacgcggagttagaag 3' |
| SEQ ID No : 27 | 5' tgacggttttttgtttattcggttattc 3' |
| SEQ ID No : 28 | 5' agtgacggttttttgtttattcggttattc 3' |
| SEQ ID No : 29 | 5' cggagggatattgcggcgtcggt 3' |
| SEQ ID No : 30 | 5' atgggaacgtaacggatga 3' |
| SEQ ID No : 31 | 5' atttaacgatttacgcggag 3' |
| SEQ ID No : 32 | 5' aaacgtacctacgcaatacgcga 3' |
| SEQ ID No : 33 | 5' cgaacgcaaacgtacctacgc 3' |
| SEQ ID No : 34 | 5' accgaacgcaaacgtacctacgca 3' |
| SEQ ID No : 35 | 5' acccaaaacgcgcttccacca 3' |
| SEQ ID No : 36 | 5' cgaataaccgaataaac 3' |
| SEQ ID No : 37 | 5' tacgcgaaaaaatac 3' |
| SEQ ID No : 38 | 5' cgccgtacgcaaccga 3' |
| SEQ ID No : 39 | 5' atttcaaaataaaaccgaacgc 3' |
| SEQ ID No : 40 | 5' acccgaccttaaaaccgaat 3' |

**Table 3 Primer sets and probes used for amplification of the methylation sites of the target gene SOX1**

| ID No : | primer sets and probes for SOX1 |
|---|---|
| SEQ ID No : 41 | 5' gcgttttttttttttcgttattggc 3' |
| SEQ ID No : 42 | 5' tgcgttttttttttttcgttattggcg 3' |
| SEQ ID No : 43 | 5' cgcggcgcgtcgttttgtta 3' |
| SEQ ID No : 44 | 5' tggaggtcgttgaggatcg 3' |
| SEQ ID No : 45 | 5' cggcggtcggcgaggagata 3' |
| SEQ ID No : 46 | 5' gcgttttcgtttcgagcgta 3' |
| SEQ ID No : 47 | 5' aggatcgagcgtaggaggaa 3' |
| SEQ ID No : 48 | 5' cgcgctatctccttcctcctacg 3' |
| SEQ ID No : 49 | 5' gccgctacgcgctatctcc 3' |
| SEQ ID No : 50 | 5' gcaacccaaacgccctcgac 3' |
| SEQ ID No : 51 | 5' cgatacgctaaacccgacccg 3' |
| SEQ ID No : 52 | 5' cgcggcgcgtcgttttgtta 3' |
| SEQ ID No : 53 | 5' cgatcctcaacgacctcca 3' |
| SEQ ID No : 54 | 5' cgatacgctaaacccgacccg 3' |
| SEQ ID No : 55 | 5' gctcgatcctcaacgacctc 3' |
| SEQ ID No : 56 | 5' acgatcgaaatcgccgtctt 3' |

**Table 4 Primer sets and probes used for amplification of the methylation sites of the target gene NKX6-1**

| ID No : | primer sets and probes for NKX6-1 |
|---|---|
| SEQ ID No : 57 | 5' tgtcgtttttcgcgtggaggg 3' |
| SEQ ID No : 58 | 5' cgtggtcgtgggatgttagc 3' |
| SEQ ID No : 59 | 5' tcggcgtggtcgtgggatgttagc 3' |
| SEQ ID No : 60 | 5' acggttttcggcgtggtcgt 3' |
| SEQ ID No : 61 | 5' ttcgggcgcgtcgagtgtt 3' |
| SEQ ID No : 62 | 5' aacatcccacgaccacgccg 3' |
| SEQ ID No : 63 | 5' acgaccacgccgaaaaccgt 3' |
| SEQ ID No : 64 | 5' acaaacaacgaaaaatacgcg 3' |
| SEQ ID No : 65 | 5' gacaaacaacgaaaaatacgcga 3' |
| SEQ ID No : 66 | 5' acgacgaaaaaaacgacgacg 3' |
| SEQ ID No : 67 | 5' cgctaccgaaaattactcg 3' |
| SEQ ID No : 68 | 5' cgaataccctccattacc 3' |
| SEQ ID No : 69 | 5' acaaacaacgaaaaatacgcg 3' |
| SEQ ID No : 70 | 5' aacactcgacgcgcccgaa 3' |

**Table 5 Primer sets and probes used for amplification of the methylation sites of the internal control genes**

| ID No : | primer sets and probes for internal control genes |
|---|---|
| SEQ ID No : 71 | 5' agggttattttgaaaagggagatat 3' |
| SEQ ID No : 72 | 5' ttttaaggggaagatgggatagaag 3' |
| SEQ ID No : 73 | 5' agaggtggggataggtattgggt 3' |
| SEQ ID No : 74 | 5' cttctatcccatcttccc 3' |
| SEQ ID No : 75 | 5' ttcattctaacccaatacct 3' |
| SEQ ID No : 76 | 5' tgttagagtaaagtatagagt 3' |
| SEQ ID No : 77 | 5' aacccaatacctatccccacctc 3' |
| SEQ ID No : 78 | 5' aacaattataaactccaaccaccaaac 3' |
| SEQ ID No : 79 | 5' actccaaccaccaaaccttcattct 3' |
| SEQ ID No : 80 | 5' accgaccccactaatacccg 3' |

**Table 6 Main components of the test composition**

| Reagents | Contents and functions |
|---|---|
| Test Mixture | containing the mixture of primers and probes of the target gene and internal control gene (a forward primer and a reverse primer) and its probe mixture |
| Master Mixture | Master mixture for PCR or Q-PCR reagent (Tag polymerase, dNDT etc.) |
| Positive Controls | Include the fragment of "Target gene" and the fragment of "Internal control gene" |

The PCR reaction is as follows: (i) activation of polymerase for 10 min at 95°C, (ii) denaturation of the DNA template for 10 seconds at 95°C and annealing/extension for 40 seconds at 60°C; and (iii) repeat the denaturation/annealing/extension cycle for 30 to 50 times.

For PCR reactions, one of the mixtures comprises the mixture of the primer sets and probes for a target gene and an internal control gene, the primer sets include a forward primer and a reverse primer and different probes are labeled with different fluorescent dyes with different wavelengths and low interference. In this example, the probe for the target gene is labeled with FAM and the probe for the internal control gene is labeled with VIC. In addition to the fluorescent dyes, all probes will also have an added quencher gene and thus the fluorescent dye will release fluorescence and be detected when the sequence of a probe is complementary and binds to the amplified sequence. The methylation status of the target genes is determined based on the Cp value (the number of cycles) and the intensity of signals, whereas the internal control genes are used for correction and detection of errors. Take the gene PAX 1 as an example, if the difference between the Cp value of PAX 1 and the Cp value of the internal control gene is less than 12, PAX 1 is significantly methylated. The same rule applies to other target genes as well.

### Example 2 Analysis of the methylation status of the target genes in different cancer cell lines

This test utilized the primer sets and probes disclosed in Table 1 to Table 4 for amplification of the methylated regions of PAX1, ZNF582, SOX1 and NKX6-1 in different cancer cell lines, and the results of the methylation status are shown in Table 7. The results indicate that methylation of PAX1, ZNF582, SOX1 and NKX6-1 was detected in cervical cancer cell line, Hela; methylation of PAX1, ZNF582, and SOX1 was detected in cervical cancer cell line, SiHa; methylation of PAX1, ZNF582, SOX1 and NKX6-1 was detected in cervical cancer cell line, CaSki; methylation of ZNF582, SOX1 and NKX6-1 was detected in cervical cancer cell line, C-33 A; methylation of PAX1, ZNF582, SOX1 and NKX6-1 was detected in colorectal cancer cell line, COLO 205; methylation of ZNF582, SOX1 and NKX6-1 was detected in colorectal cancer cell line, Caco-2; methylation of PAX1, ZNF582, SOX1 and NKX6-1 was detected in colorectal cancer cell line, HT-29. Methylation of SOX1 and NKX6-1 was detected in liver cancer cell line, HuH-7; methylation of ZNF582 and SOX1 was detected in liver cancer cell line, Mahlavu. Methylation of PAX1, ZNF582, SOX1 and NKX6-1 was not detected in lung cancer cell line, A549. Methylation of PAX1, ZNF582, SOX1 and NKX6-1 was not detected in skin cancer cell line, A-375. Methylation of ZNF582 and SOX1 was detected in ovarian cancer cell line, A2780. Methylation of PAX1 and NKX6-1was detected in breast cancer cell line, T47D; methylation of PAX1, ZNF582, and SOX1 was detected in breast cancer cell line, BT474; methylation of ZNF582, SOX1 and NKX6-1 was detected in breast cancer cell line, MDA-MB-231; methylation of ZNF582, SOX1 and NKX6-1 was detected in breast cancer cell line, ZR-75-1; methylation of ZNF582 and SOX1 was detected in breast cancer cell line, HCC1954; methylation of PAX1, ZNF582, SOX1 and NKX6-1 was detected in breast cancer cell line, MCF-7. Methylation of PAX1, ZNF582, SOX1 and NKX6-1 was detected in tongue cancer cell line, SAS. Methylation of PAX1, ZNF582, SOX1 and NKX6-1 was detected in oral cancer cell line, Ca9-22.

In summary, at least one of the four genes was found to be methylated in cervical cancer, colorectal cancer, liver cancer, ovarian cancer, breast cancer, tongue cancer, oral cancer, lung cancer, lung adenocarcinoma and skin cancer cell lines. Therefore, methylation of at least one of the PAX1, ZNF582, SOX1 and NKX6-1 gene can be used as a test indicator for cancer screening.

**Table 7 Analysis of the methylation status of the target genes in different cancer cell lines**

| | PAX1 | ZNF582 | SOX1 | NKX6-1 | Remark |
|---|---|---|---|---|---|
| Hela (CCL-2.2) | + | + | + | + | Adenocarcinoma (cervix) (HPV (18(+)) |
| SiHa | + | + | + | - | Epidermoid carcinoma (cervix) (HPV-16 (+)) |
| CaSki | + | + | + | + | Epidermoid carcinoma (cervix) (HPV-16 (+)) |
| C-33A | - | + | + | + | Carcinoma (cervix) (HPV (-)) |
| COLO 205 | + | + | + | + | Colorectal adenocarcinoma, metastatic site: ascites |
| Caco-2 | - | + | + | + | Colorectal adenocarcinoma |
| HT-29 | + | + | + | + | Colorectal adenocarcinoma |
| HuH-7 | - | - | + | + | Hepato cellular carcinoma |
| Mahlavu | - | + | + | - | Mahlavu hepatocellular carcinoma |
| A549 | - | - | - | - | Lung carcinoma |
| A-375 | - | - | - | - | Skin; malignant melanoma |
| A2780 | - | + | + | - | Ovarian carcinoma |
| T47D | + | - | - | + | Breast ductal carcinoma |
| BT474 | + | + | + | - | Breast ductal carcinoma |
| MDA-MB-231 | - | + | + | + | Breast adenocarcinoma |
| ZR-75-1 | - | + | + | + | Breast ductal carcinoma |
| HCC1954 | - | + | + | - | Breast ductal carcinoma |
| MCF-7 | + | + | + | + | Breast adenocarcinoma |
| SAS | + | + | + | + | Tongue, squamous carcinoma |
| Ca9-22 | + | + | + | + | Carcinoma; squamous cell carcinoma |

### Example 3 Analysis of the methylation status of the target genes in cervical cancer samples

The test was conducted on 279 diagnosed normal and cervical cancer samples collected in Taiwan and as shown in Table 8, 239 samples are normal (85.7%), 22 samples are CIN1 (7.9%), 2 samples are CIN2 (0.7%), 12 samples are CIN3/CIS (4.3%), and 4 samples are squamous cell carcinoma (1.4%). The DNA of these samples was extracted and then treated with bisulfite, and the primer sets and probes disclosed in Table 1 to Table 4 for amplification of the methylated regions in PAX1, ZNF582, SOX1, and NKX6-1 were used for detection. As indicated in Table 9, when compared with the normal cervical samples, using PAX1, ZNF582, SOX1, and NKX6-1 as the target gene to examine the severe cervical dysplasia samples showed 85.45-fold (95% CI = 33.95 - 215.11), 289.17-fold (95% CI = 39.20 ∼ 2133.14), 67.69-fold (95% CI = 20.55 - 223.01) and 2.56-fold (95% CI = 1.36 to 4.82) increased the occurrence of severe cervical cancer, respectively. As demonstrated in Table 10, the test sample is determined as a positive cervical cancer screening test result if either the methylated target gene or Pap smear test result is positive when the individual methylated target gene and Pap smear are used simultaneously for disease detection. When compared with the normal cervical samples, using PAX1, ZNF582, SOX1, and NKX6-1 as the target genes and combined with Pap smear to examine the severe cervical dysplasia samples indicated a 475.64-fold (95% CI = 63.94 ∼ 3538.43), 543.31-fold (95% CI = 33.11 ∼ 8914.18), 95.08-fold (95% CI = 20.55 ∼ 223.01) and 14.25-fold (95% CI = 6.12 ∼ 33.18) increased the occurrence of severe cervical cancer, respectively; moreover, the sensitivity of all groups increased when compared with a test without Pap smear (PAX1: increased from 75% to 94%, ZNF582: from 87% to 94%, SOX1: from 75% to 100%, and NKX6-1: from 50.00% to 84.78%).

**Table 8 Normal cervical samples and cervical cancer samples at different stages**

| Number of samples | Normal | CIN1 | CIN2 | CIN3/CIS | SCC |
|---|---|---|---|---|---|
| 279 | 239 (85.7%) | 22 (7.9%) | 2 (0.7%) | 12 (43%) | 4 (1.4%) |

**Table 9**

| **Target gene** | **Sensitivity** | **Specificity** | **P-value** | **Odds ratio** (**95%CI)** ^{*} |
|---|---|---|---|---|
| PAX1 | 75% | 95% | <0.0001 ^{a} | 85.45 (33.95∼215.11) |
| ZNF582 | 87% | 58% | <0.0001 ^{a} | 289.17 (39.20∼2133.14 ) |
| SOX1 | 75% | 98% | <0.0001 ^{a} | 67.69 (20.55∼223.01) |
| NKX6-1 | 50.00% | 73.05% | 0.0015 ^{a} | 2.56 (1.36∼4.82) |

**Table 10**

| **Target gene** | **Sensitivity** | **Specificity** | **P-value a** | **Odds ratio (95% CI)** ^{*} |
|---|---|---|---|---|
| PAX1 or Pap | 94% | 92% | <0.0001 ^{a} | 475.64 (63.94∼3538.43) |
| ZNF582 or Pap | 94% | 95% | <0.0001 ^{a} | 543.31 (33.11∼8914.18) |
| SOX1 or Pap | 100% | 56% | <0.0001 ^{a} | 95.08 (22.66∼398.96) |
| NKX6-1 or Pap | 84.78% | 70.13% | <0.0001 ^{a} | 14.25 (6.12∼33.18 ) |

| | | | | |
|---|---|---|---|---|
| ^{a} according to chi-square test ^{b} according to Fisher accuracy test ^{*} odds ratios (OR) of CIN3 and above (excludes CIN1 and CIN2) CIN: cervical intraepithelial neoplasia SCC: squamous cell carcinoma Pap: papanicolaou test | | | | |

### Example 4:

The test utilized 8 diagnosed normal and oral cancer smear samples, and the DNA was extracted from these samples and treated with bisulfite. The primer sets and probes disclosed in Table 1 to Table 4 for amplification of the methylation regions of PAX1, ZNF582, SOX1 and NKX6-1 were used for detection, and the methylation status of the genes is shown in Table 11. The results indicate that in oral cavity 1, no methylation of PAX1, ZNF582, SOX1, and NKX6-1 was observed; in oral cavity 2, no methylation of PAX1, ZNF582, SOX1, and NKX6-1 was observed; in oral cavity 3, methylation of PAX1 and ZNF582 was detected; in oral cavity 4, methylation of PAX1, ZNF582, SOX1, and NKX6-1 was found; in oral cavity 5, methylation of PAX1 and ZNF582 was detected; in oral cavity 6, ZNF582 is methylated; in oral cavity 7, methylation of ZNF582, SOX1, and NKX6-1 was detected; and in oral cavity 8, methylation of PAX1 and ZNF582 was detected. Hence, at least one of the four genes is significantly methylated in oral cancers at different stages and with various symptoms, and the methylation status of at least one of the four genes, PAX1, ZNF582, SOX1, and NKX6-1, can indeed be used as an indicator for oral cancer screening.

**Table 11 Analysis of the methylation status of the target genes in different oral cancer samples**

| Name | Stages and characteristics of oral cancer | PAX1 | ZNF582 | SOX1 | NKX6-1 |
|---|---|---|---|---|---|
| Oral cavity 1 | A white plaque and keratosis on the left (Leukoplakia) | - | - | - | - |
| Oral cavity 2 | A white plaque and keratosis on the left (Leukoplakia) | - | - | - | - |
| Oral cavity 3 | A white plaque and severe surface keratosis on the right (Leukoplakia), Verruciform leukoplakia | +++ | +++ | - | - |
| Oral cavity 4 | Overgrowth lesion on the left with white surrounding, Candida infection, the lesion was confirmed by histopathologic diagnosis and is free of cancer cells (Erythroleukoplakia) | +++ | +++ | +++ | +++ |
| Oral cavity 5 | Red overgrowth lesion on the left, increased white spots (Erythroleukoplakia with Oral cancer history) | +++ | +++ | - | - |
| Oral cavity 6 | Spot-like whit plaque all over the oral cavity (Leukoplakia) | - | +++ | - | - |
| Oral cavity 7 | White plaques in the oral cavity (Leukoplakia) | - | +++ | +++ | +++ |
| Oral cavity 8 | Red plaque on the right, Candida infection, carcinoma in situ (Erythroleukoplakia with Oral cancer) | +++ | +++ | - | - |

## Claims

1. A test composition features in determination of the presence of methylated CpG sequences of the genes in samples, that is, extracting gDNAfrom samples and subjecting the extracted DNA to appropriate pre-treatments and chemical reactions before using the test composition to examine the presence of gene methylation, said test composition comprising:
(1) a gene-specific primer mixture at a concentration between 20 nM and 1250 nM, said mixture is consisting of at least one forward primer and one reverse primer, the sequences of said primers are selected from the nucleotide sequences that share at least 80% homology with the sequences indicated in SEQ ID No: 1-70 or is one or more sequences containing at least 10 continuous nucleotides identical to the sequences indicated in SEQ ID No: 1-70;
(2) a nucleic acid molecule test mixture for internal control genes at a concentration between 20 nM and 1250 nM, said mixture comprises at least one forward primer and one reverse primer;
(3) a master mixture for PCR, the main ingredients of said mixture include at least polymerase, dNTPs, and Magnesium.

2. A test composition as recited in claim 1, wherein the features of the test composition are:
(1) the gene-specific primer mixture for target genes further comprises probes and the sequences of said probes are selected from one or more or complementary sequences that share at least 80% homology with the sequences indicated in SEQ ID No: 1-70 or contain at least 10 continuous nucleotides identical to the sequences indicated in SEQ ID No:1-70;
(2) the nucleic acid molecule test mixture for internal control genes further comprises the probes that are capable of detection of amplified PCR products of the internal control genes.

3. The test mixture as recited in claim 1, wherein the nucleic acid molecule test mixture for internal control genes contains forward primers and reverse primers and the sequences of said primers are selected from the nucleotide sequences that share at least 80% homology with the sequences indicated in SEQ ID No: 71-80, or is one or more sequences containing at least 10 continuous nucleotides identical to the sequences indicated in SEQ ID No: 71-80.

4. The test composition as recited in claim 2, wherein the nucleic acid molecule test mixture for internal control genes further comprises probes, wherein the sequences of the forward primers and reverse primers for the internal control genes are selected from the nucleotide sequences that share at least 80% homology with the sequences indicated in SEQ ID No: 71-80, or is one or more or complementary sequences containing at least 10 continuous nucleotides identical to the sequences indicated in SEQ ID No: 71-80.

5. The test composition as recited in claim 1, wherein the sequences of the forward primers and reverse primers for the target genes are selected from the one or more of the nucleotide sequences indicated in SEQ ID No: 1-70.

6. The target genes as recited in claim 2, wherein the sequences of the forward primers and reverse primers are selected from the one or more of the nucleotide sequences indicated in SEQ ID No: 1-70; the sequences of the probes for the target genes are selected from one or more or complementary sequences of the nucleotide sequences indicated in SEQ ID No: 71-80.

7. The internal control genes as recited in claim 4, wherein the sequences of the forward primers and reverse primers are selected from one or more of the nucleotide sequences indicated in SEQ ID No: 71-80; the sequences of the probes are selected from one or more or complementary sequences of the nucleotide sequences indicated in SEQ ID No: 71-80 .

8. The test composition as recited in claim 1, wherein the master mixture for PCR comprises a fluorescent substance that can identify the amplified PCR products or a detectable substance that can react with double-stranded DNA and produce fluorescence.

9. The test composition as recited in claim 2, wherein the probes for the target genes and internal control genes are labeled with fluorescent dyes, said fluorescent labels are selected from one of the following fluorescent dyes: FAM, HEX, TET, TAMRA, Cy3, Cy5, Cy5.5, VIC, Red610, Yellow 555, Texas Red, Yakima Yellow, BHQ-1, BHQ-2, and BHQ-3.

10. The test composition as recited in claim 1, wherein the test composition can be further used for detection of abnormal cell proliferation.

11. The test composition as recited in claim 10, wherein detection of abnormal cell proliferation includes precancerous lesions, tumor detection, detection of tumor recurrence, prediction of the effect of cancer drug, or detection of cancer outcomes.

12. The tumors as recited in claim 11, wherein the tumor is one of the following: cervical cancer, oral cancer, head and neck cancer, esophageal cancer, colorectal cancer, liver cancer, ovarian cancer, breast cancer, tongue cancer, lung cancer, lung adenocarcinoma, and skin cancer.

13. The test composition as recited in claim 2, wherein the test composition can be further used for detection of abnormal cell proliferation.

14. The test composition as recited in claim 13, wherein detection of abnormal cell proliferation includes precancerous lesions, tumor detection, detection of tumor recurrence, prediction of the effect of cancer drug, or detection of cancer outcomes.

15. The tumors as recited in claim 14, wherein the tumor is one of the following: cervical cancer, oral cancer, head and neck cancer, esophageal cancer, colorectal cancer, liver cancer, ovarian cancer, breast cancer, tongue cancer, lung cancer, lung adenocarcinoma, and skin cancer.

16. The test composition as recited in claim 1, wherein the primers for the target genes and internal control genes can be mixed together for preparation of the nucleic acid molecule test mixture in a single tube.

17. The test composition as recited in claim 2, wherein the primers and probes for the target genes and the internal control genes can be mixed together for preparation of the nucleic acid molecule test mixture in a single tube.

18. The test composition as recited in claim 1 or 2, wherein the target genes in the samples are selected from at least one or more of the following genes: PAX1, ZNF582, SOX1, and NKX6-1.

19. The test composition as recited in claim 1 or 2, wherein the internal control genes are selected from at least one or more of the following genes: Col2A, ß-Globin, GAPDH, and ß-actin.

20. The test composition as recited in claim 1 or 2, wherein the identification methods for amplified PCR products include fluorescence, sequencing, microarrays, mass spectrometer, denaturing high-performance liquid chromatography (DHPLC), pyrosequencing, or immunoassay.
